# EUROPEAN PATENT SPECIFICATION

(11) **EP 3 307 100 B1**
(45) Date of publication and mention of the grant of the patent: **02.08.2023**
(21) Application number: 16732484.7
(22) Date of filing: 08.06.2016
(51) Int. Cl.: A24F 40/42, A24F 40/48, A61M 15/06, A24F 40/10

(54) **DISPENSING MECHANISM**
AUSGABEMECHANISMUS
MÉCANISME DE DISTRIBUTION

(30) Priority: 12.06.2015 EP 15171955
(43) Date of publication of application: 18.04.2018
(73) Proprietor: Philip Morris Products S.A., 2000 Neuchâtel (CH)
(72) Inventor: PATORET, Véronique, 69170 Joux (FR)
(74) Representative: Gritschneder, Sebastian
(86) International application number: PCT/EP2016/063049
(87) International publication number: WO 2016/198459

(56) References cited:
- WO-A2-2007/000330
- WO-A2-2012/045683
- GB-A- 2 519 782
- US-A1- 2013 213 418
- US-A1- 2015 117 842

## Description

The present invention relates to dispensing mechanisms for aerosol-generating articles that preferably comprise a liquid storage portion filled with liquid and a vaporiser where the liquid is vaporised.

Such articles usually use a wick or foam impregnated of liquid to draw the liquid to the vaporiser. With such a design, several key factors of the smoking experiences cannot be correctly controlled, in particular the quantity of liquid that will be vaporised during one puff, i.e. during one draw of air.

This quantity changes according to the concentration of liquid in the foam or wick as the foam or wick may be more or less impregnated or dried. The quantity of liquid that will be vaporised during one puff is also influenced by the duration of the drawing of air as the vaporiser is activated and vaporises liquid as long as the user draws air. The quantity also depends on the age of the wick or foam as the capillary properties may change over time. Furthermore, the kind of movements and gestures of a user before drawing air have an impact on the quantity of liquid in the foam or wick. This impact is at least partially related to gravity and the filling level of the liquid storage portion, and thus causing the wick or foam of being more or less impregnated.

The quantity of nicotine delivered per puff is directly related to the quantity of liquid being vaporised. As the quantity of liquid to be vaporised cannot be determined or efficiently limited, the user experience could be different from one puff to another and from similar products to another. Another point is that, with such a system, the number of puffs that can be attributed to a full liquid storage portion cannot be well determined in advance, although it could be a strong marketing feature.

WO 2014/150131 A1 discloses an electronic smoking article comprising a liquid supply reservoir and an electrically operated valve that leads the liquid to a capillary tube with heaters around for vaporizing the liquid. A puff sensor detects the drawing of air in order to open the valve and to activate the heater. Upon conclusion of a puff, the valve may be closed to reduce the amount of liquid that is drawn back from the capillary tube because of the release of pressure. The amount of liquid to be vaporised during one draw of air may change from puff to puff and depends on pressure and duration.

US 2011/0120455 A1 discloses an aerosol dispensing device with a puff-controlled valve. The valve block is biased by a compression spring against a valve seat that keeps the valve closed. Upon a drawing of air exceeding the compression force of the spring, the valve opens. The amount of liquid to be vaporised during one draw of air may change from puff to puff and depends on pressure and duration.

US 2015/117842 A1 relates to an aerosol delivery device including a positive displacement aerosol delivery mechanism.

It would be desirable to provide an improved dispensing mechanism for aerosol-generating articles that limits the amount of liquid to be vaporised during one puff, thereby guaranteeing a minimum number of puffs attributed to a full liquid storage portion, and that is based on a technically less complex mechanism.

According to a first aspect of the invention, a dispensing mechanism for an aerosol-generating article as defined in claim 1 is provided.

According to a second aspect of the invention, an aerosol-generating article is provided, comprising a liquid storage portion configured to store pressurized liquid, a vaporiser configured to vaporise liquid, the dispensing mechanism according to the first aspect of the invention that connects the liquid storage portion to the vaporizer, and a housing having at least one air inlet and at least one air outlet, wherein the vaporiser is configured to receive liquid from the outlet of the dispensing reservoir.

According to a third aspect of the invention, an aerosol-generating system is provided, comprising the aerosol-generating article according to the second aspect of the invention, and a mouthpiece arranged to deliver the vaporised liquid from the vaporiser to a user that draws into the mouthpiece.

The expandable buffering reservoir may have a minimum volume that is expandable up to a maximum volume.

The expandable buffering reservoir and the dispensing reservoir may be configured such that the difference between the maximum volume and the minimum volume of the expandable buffering reservoir is greater or equal the constant volume of the dispensing reservoir. By doing so, the volume of the dispensing reservoir is entirely filled with liquid upon one puff.

The dispensing mechanism may further comprise a biasing member that is configured to apply a compressive force onto the volume modifier such that the expanded volume of the expandable buffering reservoir is compressed. The compression force holds the volume modifier at an initial position that corresponds to a minimum volume of the expandable buffering reservoir.

The dispensing mechanism may further comprise a dispenser connected to the outlet of the dispensing reservoir. Preferably, the dispenser is configured to open when mechanically pressed and otherwise to stay closed. More preferably, the dispenser is embodied as a deformable opening at one end of the dispensing reservoir.

The volume modifier comprises a piston that is configured to define, together with a housing in which the dispensing mechanism is arranged, a moving chamber.

The expandable buffering reservoir is coupled to one side of the piston and arranged in the moving chamber such that moving the piston longitudinally relative to the housing causes modifying the volume of the expandable buffering reservoir.

The dispensing reservoir is coupled to the other side of the piston opposite to the moving chamber, thereby causing the dispensing reservoir to be moved together with the piston longitudinally in the housing.

The piston may comprise the outlet valve connecting the outlet of the expandable buffering reservoir to the inlet of the dispensing reservoir.

The biasing member may be a compression spring attached to the piston. The compression force of the compression spring pushes the moving chamber away from a vaporiser and holds the moving chamber in an initial position. Preferably, the Resistance To Draw (RTD) is controlled by changing the characteristics of the biasing member, for example by selecting a compression spring having a compression force that corresponds to the required RTD. The RTD is an important parameter of the smoking experience. The harder the spring, the stronger the user will have to draw air to put e-liquid on the vaporiser, and therefore the bigger the RTD.

The expandable buffering reservoir is deformable. Preferably, the expandable buffering reservoir is accordion-shaped, and wherein modifying the volume of the expandable buffering reservoir comprises adjusting the length of the expandable buffering reservoir. The expandable buffering reservoir is connected to an inlet valve and an outlet valve, both valves acting as one-way valves. The inlet value allows liquid only to enter into the expandable buffering reservoir and the outlet valve only to flow out of the expandable buffering reservoir. The expandable buffering reservoir may be made from a polymer, for example a plastic polymer such as PVC or PP, which is molded accordion-shaped.

The dispensing mechanism may further comprise a chained arrangement of one or more expandable drawing reservoirs, wherein the one or more expandable drawing reservoirs are arranged between the expandable buffering reservoir and the dispensing reservoir.

Each of the one or more expandable drawing reservoirs may be connected to a neighbouring reservoir by a one-way valve in such a way that liquid only flows from the expandable buffering reservoir to the dispensing reservoir.

At least a part of the one or more expandable drawing reservoirs may be attached to a side of the piston that is opposite the expandable buffering reservoir.

Preferably, the volume modifier is configured to expand the current volume of the expandable buffering reservoir to the expanded volume upon a user drawing air into the housing in which the dispensing mechanism is mounted.

Preferably, the housing comprises at least one housing stop that is configured to press or squeeze a dispenser connected to the outlet of the dispensing reservoir when the dispensing reservoir moves longitudinally relative to the housing towards the at least one housing stop upon the volume modifier expanding the volume of the expandable buffering reservoir.

The housing may comprise at least one opening between the at least one housing stop that allows the dispensing reservoir to move so that its deformable dispenser opening is next to the at least one housing stop.

Preferably, the dispensing mechanism is arranged in an aerosol-generating article with a liquid storage portion adjoining a housing of the aerosol-generating article, at the opposite side of a vaporiser. The vaporiser is arranged to vaporise liquid released by the dispenser.

The dispensing mechanism may also be arranged in an aerosol-generating device like an e-cigarette with an electronic part connected to a battery and to an embodiment of the aerosol-generating article.

Preferably, the housing has at least one air inlet and at least one air outlet such that when a user draws air into the housing, the moving chamber acts as a sliding piston and is pushed or drawn by air towards the vaporiser and moves back upon the compression force of the biasing member.

The dispensing reservoir may be configured deformable. The dispensing reservoir may be made of silicon. Alternatively or additionally, the dispensing reservoir may have a deformable opening. The deformable opening is arranged such that the content of the dispensing reservoir, preferably a liquid aerosol-forming substrate, may be released by deforming the deformable opening. The deformable opening may be mechanically deformed or squeezed as described in more detail below. The deformable opening of the dispensing reservoir may be made of a flexible material, preferably silicon, forming a baby bottle teat valve. A baby bottle teat valve is provided as a one-way valve, configured to seal the dispensing reservoir when the content of the dispensing reservoir is not pressurized and release the content of the dispensing reservoir when the content is pressurized. The dispensing reservoir may have a volume of 0.1 to 4 microliters or 0.2 to 2 microliters or around 0.4 to 0.5 microliters. Preferably, the content of liquid aerosol-forming substrate in the dispensing reservoir is completely dispensed during one puff of a user. Thus, preferably the amount of around 0.4 to 0.5 microliters of liquid aerosol-forming substrate is dispensed during one puff of a user.

The deformable opening may alternatively be configured as a duckbill valve. A duckbill valve is configured in the shape of a duckbill and is made of a flexible material such as a flexible polymer. Thus, the deformable opening may be configured having lips, preferably elastomeric lips, in the shape of a duckbill. The lips may prevent backflow and allows forward flow. In this case, the deformable opening is configured as a one-way valve. An advantage of a duckbill valve is that the duckbill valve is self-contained, i.e. seals the content of the dispensing reservoir when the content is not pressurized. Thus, leakage of the content of the dispensing reservoir may be prevented. Also, the dispensing reservoir as a whole may be in the shape of a duckbill valve or a baby bottle teat valve. Alternatively, the deformable opening of the dispensing reservoir may be configured as a two-way valve.

### First preferred embodiment

In a first preferred embodiment, the dispensing mechanism has two reservoirs: one expandable buffering reservoir and one dispensing reservoir.

When the user draws air into the housing with a drawing force that exceeds the compression force of the biasing member, the volume modifier is drawn towards housing stops at one end of the housing. The movement of the volume modifier elongates the expandable buffering reservoir and moves the dispensing reservoir to the housing stops where the dispensing reservoir is mechanically squeezed so that the dispensing reservoir releases its content on a vaporiser that functions as a heat source for vaporising the released liquid. The increase of volume due to the elongation of the expandable buffering reservoir causes a drawing of liquid from the liquid storage portion into the expandable buffering reservoir and refills it.

When there is no air drawn into the housing or when the drawing force is below the compression force of the biasing member, the biasing member pushes back the dispensing reservoir as well as the expandable buffering reservoir away from the vaporiser. The dispensing reservoir stops releasing liquid to the vaporiser. As only the content of the dispensing reservoir is released during one puff, the quantity of liquid, and accordingly of nicotine, that is delivered per draw is determined in advance. Therefore, the number of puffs per cartridge that acts as a liquid storage portion can be easily determined.

### Second preferred embodiment

In a second preferred embodiment, the dispensing mechanism has at least three reservoirs: one expandable buffering reservoir, at least one expandable drawing reservoir and one dispensing reservoir.

When the user draws air into the housing with a drawing force that exceeds the compression force of the biasing member, the volume modifier is drawn towards housing stops. The movement of the volume modifier elongates the expandable buffering reservoir and contracts the at least one expandable drawing reservoir. The movement of the volume modifier causes moving the dispensing reservoir to the housing stops where the dispensing reservoir is mechanically squeezed so that the dispensing reservoir releases its content on a vaporiser that functions as a heat source for vaporising the released liquid. The increase of volume due to the elongation of the expandable buffering reservoir causes a drawing of liquid from the liquid storage portion into the expandable buffering reservoir and refills it. The decrease of volume due to the contraction of the expandable drawing reservoir pushes liquid from the expandable drawing reservoir into the dispensing reservoir. As the dispensing reservoir is still open, the liquid is released to the vaporiser.

When there is no air drawn into the housing or when the drawing force is below the compression force of the biasing member, the biasing member pushes back the dispensing reservoir as well as the expandable drawing reservoir and the expandable buffering reservoir away from the housing stops. The dispensing reservoir stops releasing liquid to the vaporiser. The expandable drawing reservoir and the expandable buffering reservoir return to their initial position with a minimum volume of the expandable buffering reservoir and a maximum volume of the expandable drawing reservoir.

As only the volume of the dispensing reservoir plus the volume of the expandable drawing reservoir may be released during one puff, the quantity of e-liquid, and accordingly of nicotine, that is delivered per draw is limited to a fixed maximum. Therefore, a minimum number of puffs per cartridge that acts as a liquid storage portion can be easily determined in advance.

Preferably, a secured cartridge is used as liquid storage portion that is connected to the inlet valve of the dispensing mechanism. The secured cartridge comprises a chamber, a plunger and joint, a bidirectional valve, a Luer connection, a spring, and a lipido-resistant filter. The chamber of the secured cartridge contains a liquid. The chamber may contain at least one of e-liquid and flavour, pressurized by the spring. The chamber containing the liquid and the chamber of the spring are separated by the plunger and the joint. The lipido-resistant filter, allowing air but no liquid to pass, is at one end of the secured cartridge. The lipido-resistant filter assures that the pressure is equal between the spring chamber and exterior of the secured cartridge while avoiding liquid leakage. The connection with the dispensing mechanism of an aerosol-generating article is done with a Luer connection which is a standardized technology allowing an user-friendly and leak-free connection, and includes a bidirectional valve. With the bidirectional valve, the liquid inside the secured cartridge cannot flow out when the secured cartridge is not connected to the dispensing mechanism of the aerosol-generating article. When the secured cartridge is connected, the liquid can flow through the valve in and out of the secured cartridge. This structure allows to easily fill the secured cartridge with e-liquid, makes the secured cartridge easy to plug into the aerosol-generating article due to the Luer connection, and is leak proof which is a particularly important point for nicotine based e-liquid content.

Preferably, the dispensing mechanism is used in conjunction with an e-liquid cartridge as liquid storage portion, wherein the e-liquid cartridge may be connected to and disconnected from the dispensing mechanism.

Alternatively, the dispensing mechanism may also be used in conjunction with an e-liquid reservoir as liquid storage portion, wherein the user may not separate the e-liquid reservoir from the dispensing mechanism.

A plurality of dispensing systems may be arranged within one aerosol-generating article. Each dispensing system comprises a dispensing mechanism according to an embodiment of the present invention and a separate liquid storage portion.

Two dispensing systems may be arranged within one aerosol-generating article. Such an embodiment allows a user to mix two different kinds of liquid in the same e-cigarette. The user may put e-liquid into the first liquid storage portion and a specific flavour into the second liquid storage portion.

The invention will be further described, by way of example only, with reference to the accompanying drawings in which:
Figure 1 shows a cross-sectional view of an aerosol-generating article with a dispensing mechanism according to a first preferred embodiment of the present invention;
Figure 2 shows a cross-sectional view of an aerosol-generating article with a dispensing mechanism according to a second preferred embodiment of the present invention;
Figure 3A shows a cross-sectional view of an aerosol-generating device with power supply, electronics, and a dispensing mechanism according to the first preferred embodiment of the present invention; and
Figure 3B shows a cross-sectional view of an aerosol-generating device with power supply, electronics, and a dispensing mechanism according to the second preferred embodiment of the present invention.

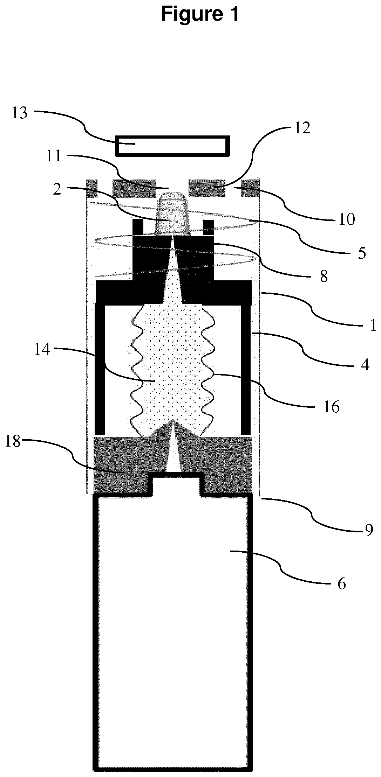

Figure 1 shows an aerosol-generating device embodied as an e-cigarette according to a first preferred embodiment of the present invention. The e-cigarette comprises an expandable buffering reservoir 16 of accordion shape attached to a volume modifier 4. The volume modifier 4 is embodied as a moving chamber in which the expandable buffering reservoir 16 is located. The volume modifier 4 is movable along the longitudinal axis of the e-cigarette. A biasing member 5 embodied as a compression spring is arranged between housing stops 12 and the volume modifier 4 to hold the volume modifier 4 in an initial position away from the housing stops 12. The expandable buffering reservoir 16 is connected with one-way valves 18, 8, with at one end a liquid storage portion 6 embodied as an e-liquid cartridge and at the other end a dispensing reservoir 2 with an opening, being able to move according to the moving of the moving chamber.

When a user draws air into the e-cigarette with a drawing force that exceeds the compression force of the biasing member 5, the air flow from the air inlet 9 to the air outlet 10 causes a moving of the volume modifier 4 towards the housing stops 12. Due to this movement, also the dispensing reservoir 2 is moved towards the housing stops 12, thereby compressing the biasing member 5. The opening of the dispensing reservoir 2 is pressed against the housing stops 12. Due to the mechanically squeezing of the opening of the dispensing reservoir 2 against the housing stops 12, the dispensing reservoir 2 releases its content on a vaporiser 13, directly or indirectly through a host material. The vaporiser 13 functions as a heat source for vaporising the released liquid. Simultaneously, due to the movement of the volume modifier 4 towards the housing stops 12, the length of the accordion-shaped expandable buffering reservoir 3 extends, and therefore increases its volume, which draws e-liquid from the liquid storage portion 6 via the one-way valve 8.

Once the user stops drawing air, the biasing member 5 pushes back the volume modifier 4 to its initial position. By doing so, the length, and therefore also the volume, of the accordion-shaped expandable buffering reservoir 3 decreases, forcing the expandable buffering reservoir 3 to expel its extra e-liquid drawn previously from the liquid storage portion 6 into the dispensing reservoir 2 via the one-way valve 7, filling anew the dispensing reservoir 2. Due to this movement, also the dispensing reservoir 2 moves away from the housing stops 12 so that the opening of the dispensing reservoir 2 is closed.

In this embodiment, the quantity of e-liquid that is released per puff on the vaporiser 13 is approximately equal to the quantity of e-liquid released from the dispensing reservoir 2. The quantity of e-liquid, and therefore of nicotine, may be accurately determined in advance, as well as the number of puffs that can be made per e-liquid cartridge. The number of puffs per e-liquid cartridge is approximately equal to the volume of the liquid storage portion 6 divided by the volume of the dispensing reservoir 2.

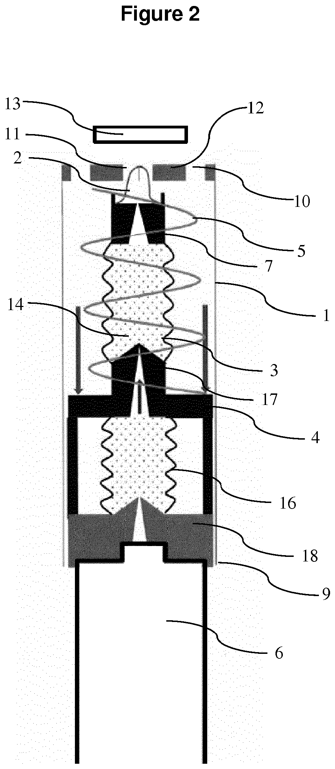

Figure 2 shows an aerosol-generating device embodied as an e-cigarette according to a second preferred embodiment of the present invention. The e-cigarette comprises an expandable buffering reservoir 16 of accordion shape attached to a volume modifier 4. The volume modifier 4 is embodied as a moving chamber in which the expandable buffering reservoir 16 is located. The volume modifier 4 is movable along the longitudinal axis of the e-cigarette. A biasing member 5 embodied as a compression spring is arranged between housing stops 12 and the volume modifier 4 to hold the volume modifier 4 in an initial position away from the housing stops 12. The expandable buffering reservoir 16 connected with one-way valves 18, 17, with at one end a liquid storage portion 6 embodied as an e-liquid cartridge and at the other end an expandable drawing reservoir 3 of accordion shape. The expandable drawing reservoir 3 is connected with one-way valves 17, 7, with at one end the expandable buffering reservoir 16 and at the other end a dispensing reservoir 2 with an opening, being able to move according to the moving of the moving chamber.

When a user draws air into the e-cigarette with a drawing force that exceeds the compression force of the biasing member 5, the air flow from the air inlet 9 to the air outlet 10 causes of a moving of the volume modifier 4 towards the vaporiser 13, thereby compressing the biasing member 5. The movement of the volume modifier 4 also moves the expandable drawing reservoir 3 and the dispensing reservoir 2 towards the housing stops 12. The dispensing reservoir 2 is then blocked by and its opening pressed against the housing stops 12. The opening of the dispensing reservoir 2 is pressed against the housing stops 12. Due to the mechanically squeezing of the opening of the dispensing reservoir 2 against the housing stops 12, the dispensing reservoir 2 releases its content on a vaporiser 13, directly or indirectly through a host material. The vaporiser 13 functions as a heat source for vaporising the released liquid. The expandable drawing reservoir 3, pressed by the moving chamber 4 and blocked by the dispensing reservoir 2, is compressed, decreasing its length, and therefore its volume, which presses its e-liquid content out of it, towards the dispensing reservoir 2 and the housing stops 12, via the one-way valve 7. Simultaneously, the length of the expandable buffering reservoir 16 extends, and therefore does its volume, which draws e-liquid from the liquid storage portion 6 via the one-way valve 18, refilling the expandable buffering reservoir 16. In this embodiment, the expandable drawing reservoir 3 will be compressed as long as the user draws air, and its content will flow into the dispensing reservoir 2 and then, due to the fact that the opening of the dispensing reservoir 2 is still open, to the vaporiser 13.

Once the user stops drawing air, the spring 5 pushes back the volume modifier 4 in its initial position. By doing so, the length, and therefore the volume, of the expandable drawing reservoir 3 increases, drawing e-liquid from the expandable buffering reservoir 16 which in turn is compressed by the action of the biasing member 5, making the expandable buffering reservoir 16 to expel e-liquid to the expandable drawing reservoir 3.

In this embodiment, the quantity of e-liquid that is released on the vaporiser 13 depends on the duration of the air drawn by the user, but has a maximum quantity which is the volume of the dispensing reservoir 2 added to the volume of the expandable drawing reservoir 3. This embodiment allows the user to easily adjust the amount of e-liquid to be vaporised per puff. The more the user draws on the e-cigarette, the bigger the puff, while at the same time, setting a fully determined maximum quantity of e-liquid, and thus a maximum amount of nicotine is vaporised per puff.

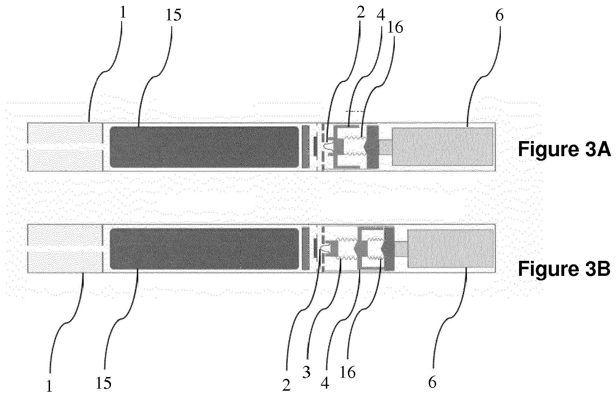

Figure 3A shows an aerosol-generating device embodied as an e-cigarette with power supply, electronics, and a dispensing mechanism according to Figure 1.

Figure 3B shows an aerosol-generating device embodied as an e-cigarette with power supply, electronics, and a dispensing mechanism according to Figure 2.

From the foregoing, it will be appreciated that specific examples of dispensing mechanisms and aerosol-generating articles, devices, and systems have been described for the purposes of illustration, but various modifications may be made without deviating from the scope of the claims. The features of embodiments described herein may be used in combination with features of other embodiments. Embodiments described herein may be implemented without all of the features ascribed to them herein.

### List of reference signs:

- 1: housing
- 2: dispensing reservoir
- 3: expandable drawing reservoir
- 4: volume modifier
- 5: biasing member
- 6: liquid storage portion
- 7: one-way valve between drawing reservoir 3 and dispensing reservoir 2
- 8: one-way valve between buffering reservoir 16 and dispensing reservoir 2
- 9: air inlet
- 10: air outlet
- 11: opening
- 12: housing stops
- 13: vaporiser
- 14: liquid
- 15: power supply and electric circuitry
- 16: expandable buffering reservoir
- 17: one-way valve between buffering reservoir 16 and drawing reservoir 3
- 18: one-way valve between cartridge 6 and buffering reservoir 16

## Claims

1. A dispensing mechanism for an aerosol-generating article, comprising:
- an expandable buffering reservoir (16) with an inlet and an outlet;
- a dispensing reservoir (2) of constant volume with an inlet and an outlet;
- an inlet valve (18) connected to the inlet of the expandable buffering reservoir (16), wherein the inlet valve (18) is configured to be connected to a liquid storage portion (6) filled with pressurized liquid such that the liquid can only flow from the liquid storage portion (6) to the expandable buffering reservoir (16);
- an outlet valve (8) connecting the outlet of the expandable buffering reservoir (16) to the inlet of the dispensing reservoir (2) such that liquid can only flow from the expandable buffering reservoir (16) to the dispensing reservoir (2); and
- a volume modifier (4) configured to modify the volume of the expandable buffering reservoir (16);
wherein expanding a current volume of the expandable buffering reservoir (16) to an expanded volume causes a drawing of liquid from the cartridge (6) to the expandable buffering reservoir (16) until the expanded volume of the expandable buffering reservoir (16) is filled up with the liquid;
wherein compressing the expanded volume of the expandable buffering reservoir (16) causes an expelling of liquid from the expandable buffering reservoir (16) to the dispensing reservoir (2) until the dispensing reservoir (2) is filled up with the liquid;
wherein the dispensing reservoir (2) is configured to release its content of liquid during one puff of a user;
wherein the volume modifier (4) comprises a piston that is configured to define, together with a housing (1) in which the dispensing mechanism is arranged, a moving chamber,
wherein the expandable buffering reservoir (16) is coupled to one side of the piston and arranged in the moving chamber such that moving the piston longitudinally relative to the housing (1) causes modifying the volume of the expandable buffering reservoir (16); and
wherein the dispensing reservoir (2) is coupled to the other side of the piston opposite to the moving chamber, thereby causing the dispensing reservoir (2) to be moved together with the piston longitudinally in the housing (1).

2. The dispensing mechanism of claim 1,
wherein the expandable buffering reservoir (16) has a minimum volume that is expandable up to a maximum volume; and
wherein the expandable buffering reservoir (16) and the dispensing reservoir (2) are configured such that the difference between the maximum volume and the minimum volume of the expandable buffering reservoir (16) is greater or equal the constant volume of the dispensing reservoir (2).

3. The dispensing mechanism of claim 1 or claim 2, further comprising:
- a biasing member (5) configured to apply a compressive force onto the volume modifier (4) such that the expanded volume of the expandable buffering reservoir (16) is compressed.

4. The dispensing mechanism of any of claims 1 to 3, further comprising:
- a dispenser connected to the outlet of the dispensing reservoir (2), wherein the dispenser is configured to open when mechanically pressed and otherwise to stay closed.

5. The dispensing mechanism of any of claims 1 to 4, wherein the dispensing reservoir comprises a deformable opening and wherein the deformable opening of the dispensing reservoir is configured as a flexible baby bottle teat valve or flexible duckbill valve.

6. The dispensing mechanism of any of claims 1 to 5,
wherein the dispensing reservoir is configured deformable.

7. The dispensing mechanism of any one of claims 1 to 6,
wherein the dispensing reservoir is made from silicon.

8. The dispensing mechanism of any one of claims 1 to 7,
wherein dispensing reservoir has a volume of 0.4 to 0.5 microliters.

9. The dispensing mechanism of any of claims 1 to 8,
wherein the piston comprises the outlet valve (8) connecting the outlet of the expandable buffering reservoir (16) to the inlet of the dispensing reservoir (2).

10. The dispensing mechanism of any of claims 3 to 9, wherein the biasing member (5) is a compression spring attached to the piston.

11. The dispensing mechanism of any of claims 1 to 10, wherein the expandable buffering reservoir (16) is accordion-shaped, and wherein modifying the volume of the expandable buffering reservoir (16) comprises adjusting the length of the expandable buffering reservoir (16).

12. The dispensing mechanism of any of claims 1 to 11, further comprising a chained arrangement of one or more expandable drawing reservoirs (3), wherein the one or more expandable drawing reservoirs (3) are arranged between the expandable buffering reservoir (16) and the dispensing reservoir (2).

13. The dispensing mechanism of claim 12, wherein each of the one or more expandable drawing reservoirs (3) is connected to a neighbouring reservoir by a one-way valve in such a way that liquid only flows from the expandable buffering reservoir (16) to the dispensing reservoir (2).

14. An aerosol-generating article, comprising:
- a liquid storage portion (6) configured to store pressurized liquid;
- a vaporiser (13) configured to vaporise liquid;
- the dispensing mechanism of any of claims 1 to 13 connecting the liquid storage portion (6) to the vaporiser (13); and
- a housing (1) having at least one air inlet (9) and at least one air outlet (10); wherein the vaporiser (13) is configured to receive liquid from the outlet of the dispensing reservoir (2).

15. The aerosol-generating article of claim 14, wherein the volume modifier (4) is configured to expand the current volume of the expandable buffering reservoir (16) to the expanded volume upon a user drawing air into the housing (1).

16. The aerosol-generating article of claim 15, wherein the housing (1) comprises at least one housing stop (12) that is configured to press or squeeze a dispenser connected to the outlet of the dispensing reservoir (3) when the dispensing reservoir moves longitudinally relative to the housing (1) towards the at least one housing stop (12) upon the volume modifier (4) expanding the volume of the expandable buffering reservoir (16).

17. An aerosol-generating system, comprising:
- the aerosol-generating article of any of claims 14 to 16,
- a mouthpiece arranged to deliver the vaporised liquid from the vaporiser (13) to a user that draws into the mouthpiece.

## Patentansprüche

1. Abgabemechanismus für einen aerosolerzeugenden Artikel, umfassend:
- einen expandierbaren Puffervorratsbehälter (16) mit einem Einlass und einem Auslass;
- einen Abgabevorratsbehälter (2) mit konstantem Volumen mit einem Einlass und einem Auslass;
- ein mit dem Einlass des expandierbaren Puffervorratsbehälters (16) verbundenes Einlassventil (18), wobei das Einlassventil (18) für die Verbindung mit einem Flüssigkeitsspeicherteil (6) ausgelegt ist, der mit druckbeaufschlagter Flüssigkeit gefüllt ist, sodass die Flüssigkeit nur von dem Flüssigkeitsspeicherteil (6) zu dem expandierbaren Puffervorratsbehälter (16) fließen kann;
- ein den Auslass des expandierbaren Puffervorratsbehälters (16) mit dem Einlass des Abgabevorratsbehälters (2) verbindendes Auslassventil (8), sodass die Flüssigkeit nur von dem expandierbaren Puffervorratsbehälter (16) zu dem Abgabevorratsbehälter (2) fließen kann; und
- einen Volumenmodifizierer (4), der zur Modifizierung des Volumens des expandierbaren Puffervorratsbehälters (16) ausgelegt ist;
wobei das Expandieren eines aktuellen Volumens des Puffervorratsbehälters (16) auf ein expandierbares Volumen ein Ansaugen von Flüssigkeit aus der Patrone (6) in den expandierbaren Puffervorratsbehälter (16) bewirkt, bis das expandierbare Volumen des Puffervorratsbehälters (16) mit der Flüssigkeit aufgefüllt ist;
wobei das Verdichten des expandierbaren Volumens des expandierbaren Puffervorratsbehälters (16) ein Ausstoßen von Flüssigkeit aus dem expandierbaren Puffervorratsbehälter (16) in den Abgabevorratsbehälter (2) bewirkt, bis der Abgabevorratsbehälter (2) mit der Flüssigkeit aufgefüllt ist;
wobei der Abgabevorratsbehälter (2) für die Freigabe seines Flüssigkeitsinhalts während eines Zuges eines Benutzers ausgelegt ist;
wobei der Volumenmodifizierer (4) einen Kolben aufweist, der zur Definition einer beweglichen Kammer zusammen mit einem Gehäuse (1) ausgelegt ist, in dem der Abgabemechanismus angeordnet ist,
wobei der expandierbare Puffervorratsbehälter (16) mit einer Seite des Kolbens gekoppelt und derart in der beweglichen Kammer angeordnet ist, dass eine Bewegung des Kolbens in Längsrichtung relativ zu dem Gehäuse (1) eine Änderung des Volumens des expandierbaren Puffervorratsbehälters (16) bewirkt; und
wobei der Abgabevorratsbehälter (2) mit der anderen, der beweglichen Kammer gegenüberliegenden Seite des Kolbens gekoppelt ist, sodass der Abgabevorratsbehälter (2) zusammen mit dem Kolben in Längsrichtung in dem Gehäuse (1) bewegt wird.

2. Abgabemechanismus nach Anspruch 1,
wobei der expandierbare Puffervorratsbehälter (16) ein Mindestvolumen aufweist, das bis zu einem Maximalvolumen expandierbar ist; und
wobei der expandierbare Puffervorratsbehälter (16) und der Abgabevorratsbehälter (2) derart ausgelegt sind, dass die Differenz zwischen dem Maximalvolumen und dem Mindestvolumen des expandierbaren Puffervorratsbehälters (16) größer als oder gleich dem konstanten Volumen des Abgabevorratsbehälters (2) ist.

3. Abgabemechanismus nach Anspruch 1 oder Anspruch 2, ferner umfassend:
- ein Vorspannelement (5), das zum Aufbringen einer Druckkraft auf den Volumenmodifizierer (4) ausgelegt ist, sodass das expandierbare Volumen des Puffervorratsbehälters (16) verdichtet wird.

4. Abgabemechanismus nach einem der Ansprüche 1 bis 3, ferner umfassend:
- einen mit dem Auslass des Abgabevorratsbehälters (2) verbundenen Spender, wobei der Spender zum Öffnen bei mechanischem Druck und ansonsten zum Geschlossenbleiben ausgelegt ist.

5. Abgabemechanismus nach einem der Ansprüche 1 bis 4, wobei der Abgabevorratsbehälter eine verformbare Öffnung aufweist und
wobei die verformbare Öffnung des Abgabevorratsbehälters als flexibles Babyflaschensaugerventil oder flexibles Entenschnabelventil ausgelegt ist.

6. Abgabemechanismus nach einem der Ansprüche 1 bis 5,
wobei der Abgabevorratsbehälter verformbar auslegt ist.

7. Abgabemechanismus nach einem der Ansprüche 1 bis 6,
wobei der Abgabevorratsbehälter aus Silikon hergestellt ist.

8. Abgabemechanismus nach einem der Ansprüche 1 bis 7,
wobei der Abgabevorratsbehälter ein Volumen von 0,4 bis 0,5 Mikroliter aufweist.

9. Abgabemechanismus nach einem der Ansprüche 1 bis 8,
wobei der Kolben das den Auslass des expandierbaren Puffervorratsbehälters (16) mit dem Einlass des Abgabevorratsbehälters (2) verbindende Auslassventil (8) aufweist.

10. Abgabemechanismus nach einem der Ansprüche 3 bis 9, wobei das Vorspannelement (5) eine an dem Kolben angebrachte Druckfeder ist.

11. Abgabemechanismus nach einem der Ansprüche 1 bis 10, wobei der expandierbare Puffervorratsbehälter (16) akkordeonförmig ist, und wobei das Modifizieren des Volumens des expandierbaren Puffervorratsbehälters (16) das Einstellen der Länge des expandierbaren Puffervorratsbehälters (16) umfasst.

12. Abgabemechanismus nach einem der Ansprüche 1 bis 11, ferner umfassend eine verkettete Anordnung von einem oder mehreren expandierbaren Ziehvorratsbehältern (3), wobei der eine oder die mehreren expandierbaren Ziehvorratsbehälter (3) zwischen dem expandierbaren Puffervorratsbehälter (16) und dem Abgabevorratsbehälter (2) angeordnet sind.

13. Abgabemechanismus nach Anspruch 12, wobei jeder der expandierbaren Ziehvorratsbehälter (3) über ein Einwegventil mit einem benachbarten Vorratsbehälter verbunden ist, sodass Flüssigkeit nur vom expandierbaren Puffervorratsbehälter (16) zu dem Abgabevorratsbehälter (2) fließt.

14. Aerosolerzeugender Artikel, umfassend:
- einen zum Speichern von druckbeaufschlagter Flüssigkeit ausgelegten Flüssigkeitsspeicherteil (6);
- einen zum Verdampfen von Flüssigkeit ausgelegten Verdampfer (13);
- der Abgabemechanismus nach einem der Ansprüche 1 bis 13, der den Flüssigkeitsspeicherteil (6) mit dem Verdampfer (13) verbindet; und
- ein Gehäuse (1), das wenigstens einen Lufteinlass (9) und wenigstens einen Luftauslass (10) aufweist;
wobei der Verdampfer (13) zum Aufnehmen von Flüssigkeit aus dem Auslass des Abgabevorratsbehälters (2) ausgelegt ist.

15. Aerosolerzeugender Artikel nach Anspruch 14, wobei der Volumenmodifizierer (4) so ausgelegt ist, dass er das aktuelle Volumen des Puffervorratsbehälters (16) auf das expandierbare Volumen erweitert, wenn ein Benutzer Luft in das Gehäuse (1) zieht.

16. Aerosolerzeugender Artikel nach Anspruch 15, wobei das Gehäuse (1) wenigstens einen Gehäuseanschlag (12) aufweist, der ausgelegt ist, einen mit dem Auslass des Abgabevorratsbehälters (3) verbundenen Spender zu drücken oder zu quetschen, wenn sich der Abgabevorratsbehälter in Längsrichtung relativ zu dem Gehäuse (1) in Richtung des wenigstens einen Gehäuseanschlags (12) bewegt, wenn der Volumenmodifizierer (4) das Volumen des expandierbaren Puffervorratsbehälters (16) expandiert.

17. Aerosolerzeugungssystem, aufweisend:
- den aerosolerzeugenden Artikel nach einem der Ansprüche 14 bis 16,
- ein Mundstück, das zur Abgabe der verdampften Flüssigkeit aus dem Verdampfer (13) an einen an dem Mundstück ziehenden Benutzer angeordnet ist.

## Revendications

1. Mécanisme de distribution pour un article de génération d'aérosol, comprenant :
- un réservoir tampon extensible (16) avec une entrée et une sortie ;
- un réservoir de distribution (2) de volume constant avec une entrée et une sortie ;
- un clapet d'entrée (18) raccordé à l'entrée du réservoir tampon extensible (16), dans lequel le clapet d'entrée (18) est configuré pour être raccordé à une partie de stockage de liquide (6) remplie de liquide sous pression de telle sorte que le liquide ne peut s'écouler que de la partie de stockage de liquide (6) vers le réservoir tampon extensible (16) ;
- un clapet de sortie (8) raccordant la sortie du réservoir tampon extensible (16) à l'entrée du réservoir de distribution (2) de telle sorte que le liquide ne peut s'écouler que du réservoir tampon extensible (16) vers le réservoir de distribution (2) ; et
- un modificateur de volume (4) configuré pour modifier le volume du réservoir tampon extensible (16) ;
dans lequel l'extension d'un volume actuel du réservoir tampon extensible (16) jusqu'à un volume étendu provoque une aspiration de liquide de la cartouche (6) vers le réservoir tampon extensible (16) jusqu'à ce que le volume étendu du réservoir tampon extensible (16) soit rempli avec le liquide ;
dans lequel la compression du volume étendu du réservoir tampon extensible (16) provoque une expulsion de liquide du réservoir tampon extensible (16) vers le réservoir de distribution (2) jusqu'à ce que le réservoir de distribution (2) soit rempli avec le liquide ;
dans lequel le réservoir de distribution (2) est configuré pour libérer son contenu de liquide pendant une bouffée d'un utilisateur ;
dans lequel le modificateur de volume (4) comprend un piston qui est configuré pour définir, conjointement avec un logement (1) dans lequel le mécanisme de distribution est agencé, une chambre mobile,
dans lequel le réservoir tampon extensible (16) est couplé à un côté du piston et agencé dans la chambre mobile de telle sorte que le déplacement du piston longitudinalement par rapport au logement (1) provoque la modification du volume du réservoir tampon extensible (16) ; et
dans lequel le réservoir de distribution (2) est couplé à l'autre côté du piston opposé à la chambre mobile, provoquant ainsi le déplacement du réservoir de distribution (2) avec le piston longitudinalement dans le logement (1).

2. Mécanisme de distribution selon la revendication 1,
dans lequel le réservoir tampon extensible (16) a un volume minimal qui est extensible jusqu'à un volume maximal ; et
dans lequel le réservoir tampon extensible (16) et le réservoir de distribution (2) sont configurés de telle sorte que la différence entre le volume maximal et le volume minimal du réservoir tampon extensible (16) est supérieure ou égale au volume constant du réservoir de distribution (2) .

3. Mécanisme de distribution selon la revendication 1 ou la revendication 2, comprenant en outre :
- un élément de sollicitation (5) configuré pour appliquer une force de compression sur le modificateur de volume (4) de telle sorte que le volume étendu du réservoir tampon extensible (16) est comprimé.

4. Mécanisme de distribution selon l'une quelconque des revendications 1 à 3, comprenant en outre :
- un distributeur raccordé à la sortie du réservoir de distribution (2), dans lequel le distributeur est configuré pour s'ouvrir lorsqu'il est pressé mécaniquement et pour rester autrement fermé.

5. Mécanisme de distribution selon l'une quelconque des revendications 1 à 4, dans lequel le réservoir de distribution comprend une ouverture déformable et
dans lequel l'ouverture déformable du réservoir de distribution est configurée comme un clapet à tétine de biberon flexible ou un clapet à bec de canard flexible.

6. Mécanisme de distribution selon l'une quelconque des revendications 1 à 5,
dans lequel le réservoir de distribution est configuré déformable.

7. Mécanisme de distribution selon l'une quelconque des revendications 1 à 6,
dans lequel le réservoir de distribution est composé de silicium.

8. Mécanisme de distribution selon l'une quelconque des revendications 1 à 7,
dans lequel le réservoir de distribution a un volume de 0,4 à 0,5 microlitre.

9. Mécanisme de distribution selon l'une quelconque des revendications 1 à 8,
dans lequel le piston comprend le clapet de sortie (8) raccordant la sortie du réservoir tampon extensible (16) à l'entrée du réservoir de distribution (2).

10. Mécanisme de distribution selon l'une quelconque des revendications 3 à 9, dans lequel l'élément de sollicitation (5) est un ressort de compression fixé au piston.

11. Mécanisme de distribution selon l'une quelconque des revendications 1 à 10, dans lequel le réservoir tampon extensible (16) est en forme d'accordéon, et dans lequel la modification du volume du réservoir tampon extensible (16) comprend le réglage de la longueur du réservoir tampon extensible (16).

12. Mécanisme de distribution selon l'une quelconque des revendications 1 à 11, comprenant en outre un agencement en chaîne d'un ou de plusieurs réservoirs d'aspiration extensibles (3), dans lequel les un ou plusieurs réservoirs d'aspiration extensibles (3) sont agencés entre le réservoir tampon extensible (16) et le réservoir de distribution (2).

13. Mécanisme de distribution selon la revendication 12, dans lequel chacun des un ou plusieurs réservoirs d'aspiration extensibles (3) est raccordé à un réservoir voisin par une clapet unidirectionnel de telle sorte que le liquide ne s'écoule que du réservoir tampon extensible (16) vers le réservoir de distribution (2).

14. Article de génération d'aérosol, comprenant :
- une partie de stockage de liquide (6) configurée pour stocker du liquide sous pression ;
- un vaporisateur (13) configuré pour vaporiser du liquide ;
- le mécanisme de distribution selon l'une quelconque des revendications 1 à 13 raccordant la partie de stockage de liquide (6) au vaporisateur (13) ; et
- un logement (1) ayant au moins une entrée d'air (9) et au moins une sortie d'air (10) ;
dans lequel le vaporisateur (13) est configuré pour recevoir du liquide depuis la sortie du réservoir de distribution (2).

15. Article de génération d'aérosol selon la revendication 14, dans lequel le modificateur de volume (4) est configuré pour étendre le volume actuel du réservoir tampon extensible (16) jusqu'au volume étendu lorsqu'un utilisateur aspire de l'air dans le logement (1).

16. Article de génération d'aérosol selon la revendication 15, dans lequel le logement (1) comprend au moins une butée de logement (12) qui est configurée pour presser ou serrer un distributeur raccordé à la sortie du réservoir de distribution (3) lorsque le réservoir de distribution se déplace longitudinalement par rapport au logement (1) vers l'au moins une butée de logement (12) lorsque le modificateur de volume (4) étend le volume du réservoir tampon extensible (16).

17. Système de génération d'aérosol, comprenant :
- un article de génération d'aérosol selon l'une quelconque des revendications 14 à 16,
- un embout buccal agencé pour fournir le liquide vaporisé depuis le vaporisateur (13) à un utilisateur qui aspire dans l'embout buccal.
